(19) Europäisches Patentamt
European Patent Office
Office européen des brevets

(11) Veröffentlichungsnummer: **0 378 048 A1**

(12) # EUROPÄISCHE PATENTANMELDUNG

(21) Anmeldenummer: **89810955.8**

(22) Anmeldetag: **14.12.89**

(51) Int. Cl.5: **C08G 65/32, C08G 65/26, D06P 1/613**

(30) Priorität: **23.12.88 CH 4771/88**

(43) Veröffentlichungstag der Anmeldung: **18.07.90 Patentblatt 90/29**

(84) Benannte Vertragsstaaten: **AT BE CH DE ES FR GB IT LI NL SE**

(71) Anmelder: **CIBA-GEIGY AG**
**Klybeckstrasse 141**
**CH-4002 Basel(CH)**

(72) Erfinder: **Töpfl, Rosemarie**
**Dorneckstrasse 68**
**CH-4143 Dornach(CH)**
Erfinder: **Merz, Jürg, Dr.**
**Langgartenstrasse 51**
**CH-4105 Biel-Benken(CH)**

(54) **Styroloxid-Anlagerungsprodukte.**

(57) Anlagerungsprodukte von Styroloxid an Polyalkylenglykolether der Formel

(1) $R\text{-}O\{Alkylen\text{-}O\}_m H$

ihre saure Ester und deren Salze,
worin R einen aliphatischen Rest mit mindestens 4 Kohlenstoffatomen bedeutet,
"Alkylen" für einen Alkylenrest von 2 oder 3 Kohlenstoffatomen steht und
m eine Zahl von 1 bis 100 ist.

Diese Produkte stellen eine neue Klasse von nichtionogenen oder anionischen Tensiden dar, welche als Textilveredlungsmittel, insbesondere als Emulgiermittel, Netzmittel, Schaumdämpfungsmittel oder Färbereihilfsmittel verwendet werden.

EP 0 378 048 A1

# Styroloxid-Anlagerungsprodukte

Die vorliegende Erfindung betrifft neue Styroloxid-Anlagerungsprodukte sowie ihre Verwendung als Textilveredlungsmittel, insbesondere als Emulgiermittel, Netzmittel, Schaumdämpfungsmittel oder Färbereihilfsmittel.

Die erfindungsgemässen Styroloxidprodukte sind dadurch gekennzeichnet, dass sie Anlagerungsprodukte von Styroloxid an Polyalkylenglykolether der Formel

(1) $R\text{-}O\text{-}(\text{Alkylen-}O)_m\text{-}H$

und ihre sauren Ester und deren Salze darstellen.

In Formel (1) bedeutet R einen aliphatischen Rest mit mindestens 4 Kohlenstoffatomen, vorzugsweise mit mindestens 6 Kohlenstoffatomen. "Alkylen" steht für einen Alkylenrest von 2 oder 3 Kohlenstoffatomen wie Ethylen oder Propylen oder deren Kombinationen. m bedeutet eine Zahl von 1 bis 100, vorteilhafterweise 2 bis 100 und vorzugsweise 4 bis 80.

Der Substituent R stellt vorteilhafterweise den Kohlenwasserstoffrest eines ungesättigten oder gesättigten aliphatischen Monoalkohols mit 6 bis 24 Kohlenstoffatomen dar. Der Kohlenwasserstoffrest kann geradkettig oder verzweigt sein. Vorzugsweise bedeutet R einen Alkyl- oder Alkenylrest mit 8 bis 22 Kohlenstoffatomen.

Als aliphatische gesättigte Monoalkohole können natürliche Alkohole, wie z.B. Laurylalkohol, Myristylalkohol, Cetylalkohol, Stearylalkohol, Arachidylalkohol oder Behenylalkohol, sowie synthetische Alkohole, z.B. Oxo-Alkohole wie insbesondere 2-Ethylbutanol, 2-Methylpentanol, 5-Methylheptan-3-ol, 2-Ethyl-hexanol, 1,1,3,3-Tetramethylbutanol, Octan-2-ol, Isononylalkohol, Trimethylhexanol, Trimethyl-nonylalkohol, Decanol, $C_9$-$C_{11}$-Oxoalkohol, Tridecylalkohol, Isotridecanol oder lineare primäre Alkohole (Alfole) mit 8 bis 18 Kohlenstoffatomen in Betracht kommen. Einige Vertreter der Alfole sind Alfol (8-10), Alfol (9-11), Alfol (10-14), Alfol (12-13) oder Alfol (16-18), ("Alfol" ist ein eingetragenes Warenzeichen).

Ungesättigte aliphatische Monoalkohole sind beispielsweise Dodecenylalkohol, Hexadecenylalkohol oder Oleylalkohol.

Die Alkoholreste können einzeln oder in Form von Gemischen von zwei oder mehreren Komponenten vorhanden sein, wie z.B. Mischungen von Alkyl-und/oder Alkenylgruppen, die sich von Soja-Fettsäuren, Palmkernfettsäuren oder Talg-Oelen ableiten.

$(\text{Alkylen-}O)_m$-Ketten sind bevorzugt vom Ethylenglykol-, Propylenethylenglykol- oder Ethylenpropylenglykol-Typus; besonders bevorzugt ist erstere.

m ist bevorzugt 4 bis 40.

Die Styroloxid-Anlagerungsprodukte werden hergestellt, indem man zuerst den aliphatischen Monoalkohol mit 1 bis 100 Mol Alkylenoxid (Ethylenoxid und/oder Propylenoxid) verethert und dann 1 Mol Styroloxid an den erhaltenen Polyalkylenglykolether anlagert. Die Veretherung des Monoalkohols wird nach an sich bekannten Methoden durchgeführt, wobei Ethylenoxid oder Propylenoxid oder abwechselweise Ethylenoxid und Propylenoxid oder Mischungen von Ethylenoxid und Propylenoxid verwendet werden. Die Styroloxidanlagerung erfolgt zweckmässigerweise in einem praktisch wasserfreien oder wasserarmen Medium bei einer Temperatur von 40 bis 90 °C mit oder ohne Druck und in Gegenwart eines sauren Katalysators, wie z.B. Bortrifluoretherat, p-Toluolsulfonsäure, Phosphorsäure oder vor allem Schwefelsäure. Die Säure wird dann durch Neutralisieren und Filtrieren entfernt.

Als Beispiele für zur Styroloxidanlagerung benötigte Polyalkylenglykolether, die der Formel (1) entsprechen, seien im einzelnen genannt:

- Anlagerungsprodukt von 8 Mol Ethylenoxid an 1 Mol $C_9$-$C_{11}$-Oxoalkohol
- Anlagerungsprodukt von 2 Mol Ethylenoxid an 1 Mol Butanol
- Anlagerungsprodukt von 2,5 Mol Ethylenoxid an 1 Mol $C_9$-$C_{11}$-Oxoalkohol
- Anlagerungsprodukt von 4 Mol Ethylenoxid an 1 Mol $C_9$-$C_{11}$-Oxoalkohol
- Anlagerungsprodukt von 5 Mol Ethylenoxid an 1 Mol $C_9$-$C_{11}$-Oxoalkohol
- Anlagerungsprodukt von 35 Mol Ethylenoxid an 1 Mol Stearylalkohol
- Anlagerungsprodukt von 36 Mol Ethylenoxid an 1 Mol Stearylalkohol
- Anlagerungsprodukt von 18 Mol Ethylenoxid an 1 Mol $C_{12}$-$C_{18}$-Fettalkoholgemisch (Cetalol 50-55)
- Anlagerungsprodukt von 10 Mol Ethylenoxid an 1 Mol 2-Ethylhexanol
- Anlagerungsprodukt von 4 Mol Ethylenoxid und 12 Mol Propylenoxid an 1 Mol $C_9$-$C_{11}$-Oxoalkohol
- Anlagerungsprodukt von 8 Mol Ethylenoxid und 12 Mol Propylenoxid an 1 Mol $C_{12}$-$C_{13}$-Fettalkohol
- Anlagerungsprodukt von 6 Mol Ethylenoxid und 6 Mol Propylenoxid an 1 Mol $C_9$-$C_{11}$-Oxoalkohol
- Anlagerungsprodukt von 3 Mol Propylenoxid und 3 Mol Ethylenoxid an 1 Mol Dodecylalkohol
- Anlagerungsprodukt von 18 Mol Ethylenoxid an 1 Mol Cetylalkohol

- Anlagerungsprodukt von 3 Mol Ethylenoxid an 1 Mol Isotridecylalkohol
- Anlagerungsprodukt von 9 Mol Ethylenoxid an 1 Mol Tridecylalkohol
- Anlagerungsprodukt von 80 Mol Ethylenoxid an 1 Mol Oleylalkohol
- Anlagerungsprodukt von 10 Mol Ethylenoxid an 1 Mol Tridecylalkohol
- Anlagerungsprodukt von 20 Mol Ethylenoxid an 1 Mol Oleylalkohol
- Anlagerungsprodukt von 3 Mol Ethylenoxid an 1 Mol Laurylalkohol,
- Anlagerungsprodukt von 2, 4, 6 oder 15 Mol Ethylenoxid an 1 Mol Nonylakolhol sowie auch Ethylenglykolmonobutylether.

Die sauren Ester können je nach dem Säurerest in Form von Monoester, Diester oder Halbester und als freie Säuren oder vorzugsweise als Salze z.B. Alkalimetallsalze oder Erdalkalimetallsalze oder Ammoniumsalze vorliegen. Als Alkalimetallsalze seien insbesondere die Natrium-, Kalium-oder Lithiumsalze, als Erdalkalimetallsalze die Magnesium- oder Calciumsalze und als Ammoniumsalze die Ammonium-, Dimethylammonium-, Trimethylammonium-, Monoethanolammonium-, Diethanolammonium- und Triethanolammoniumsalze genannt. Vorzugsweise werden die sauren Ester als Ammoniumsalze hergestellt. Mono- oder Diethanolammoniumsalze können noch weiter mit 1 bis 25 Oxyethylen-Einheiten verethert sein.

Die sauren Ester werden hergestellt, indem man das erfindungsgemässe Styroloxidanlagerungsprodukt mit einer mindestens zweibasischen Sauerstoffsäure umsetzt und gewünschtenfalls den erhaltenen sauren Ester in die obengenannten Salze überführt.

Als mehrbasische Sauerstoffsäuren für die Bildung der sauren Ester können gegebenenfalls sulfonierte, organische, vorzugsweise aliphatische Dicarbonsäuren von 3 bis 6 Kohlenstoffatomen, wie z.B. Maleinsäure, Malonsäure, Bernsteinsäure oder Sulfobernsteinsäure, oder mehrbasische anorganische Sauerstoffsäuren, wie z.B. Schwefelsäure oder Orthophosphorsäure dienen. Anstelle der Säuren können deren funktionelle Derivate wie Säureanhydride, Säurehalogenide oder Säureamide verwendet werden. Als Beispiele dieser funktionellen Derivate seien Maleinsäureanhydrid, Phosphorpentoxid, Chlorsulfonsäure und Sulfaminsäure genannt. Die Phosphorsäureester entstehen zweckmässigerweise als Gemische eines Mono- und Diesters.

Die Veresterung wird in der Regel durch einfaches Vermischen der Reaktionspartner unter Erwärmen, zweckmässig auf eine Temperatur zwischen 50° und 100°C, durchgeführt. Die zunächst entstehenden, freien Säuren können anschliessend in die entsprechenden Alkalimetall- oder Ammoniumsalze übergeführt werden. Die Ueberführung in die Salze erfolgt auf übliche Weise durch Zugabe von Basen, wie z.B. Ammoniak, Monoethanolamin, Triethanolamin oder Alkalimetallhydroxyde, z.B. Natrium- oder Kaliumhydroxyd. Gemäss einer besonders bevorzugten Ausführungsart werden saure Schwefelsäureester in Form ihrer Ammoniumsalze direkt hergestellt, indem man die Styroloxidanlagerungsprodukte, zweckmässig in Gegenwart von Harnstoff, mit Sulfaminsäure erwärmt.

Praktisch wichtige Styroloxidanlagerungsprodukte entsprechen der Formel

$$(2) \qquad R_1-O-(CH_2CH_2O)_{m_1}-\underset{Y_1}{CH}-\underset{Y_2}{CH}-OH$$

worin
$R_1$ Alkyl oder Alkenyl je mit 8 bis 22 Kohlenstoffatomen,
von $Y_1$ und $Y_2$ eines Phenyl und das andere Wasserstoff und
$m_1$ 4 bis 80 bedeuten.

Bevorzugte mit einer anorganischen oder organischen Säure hergestellte saure Ester entsprechen der Formel

$$(3) \qquad R_1-O-(CH_2CH_2O)_{m_1}-\underset{Y_1}{CH}-\underset{Y_2}{CH}-O-X$$

oder der Formel

$$(4) \qquad R_1-O-(CH_2CH_2O)_{n_1}-(\underset{Z_1}{CH}-\underset{Z_2}{CH}O)_{n_2}-\underset{Y_1}{CH}-\underset{Y_2}{CH}-O-X$$

worin $R_1$, $Y_1$, $Y_2$ und $m_1$ die angegebene Bedeutung haben,

von $Z_1$ und $Z_2$ eines Methyl und das andere Wasserstoff,
X den Maleinsäure-, Sulfobernsteinsäure-, Schwefelsäure- oder Phosphorsäurerest und die Summe von $n_1 + n_2$ 4 bis 30, vorzugsweise 6 bis 18 bedeuten.

Besonders bevorzugte saure Ester der Formeln (3) und (4) enthalten entweder eine Maleinsäureestergruppe oder eine Schwefelsäureestergruppe, die vorzugsweise in Form ihrer Alkalimetallsalze oder Ammoniumsalze vorliegen.

Erfindungsgemässe Styroloxidanlagerungsprodukte eignen sich für die verschiedensten Zwecke in der Textilapplikation, wie z.B. Vorbehandlung, Färben oder Ausrüstung. Nichtionogene unveresterte Produkte finden insbesondere Verwendung als Hilfsmittel beim Färben von wollhaltigen Fasermaterialien mit anionischen Farbstoffen oder Farbstoffgemischen. Die entsprechenden sauren Ester, besonders die Dicarbonsäure-Halbester oder Schwefelsäureester hingegen werden vorallem als Netzmittel und Schaumdämpfungsmittel für wässrige Systeme, insbesondere beim Färben von natürlichem oder synthetischem Fasermaterial und in erster Linie beim Färben von Cellulose-Textilmaterialien, Polyesterfasern oder natürlichen oder synthetischen Polyamidfasermaterialien verwendet. Sowohl als nichtionogene Produkte als auch als saure Ester verbessern erfindungsgemässe Styroloxidanlagerungsprodukte in den beschriebenen Formulierungen das Aufziehvermögen der Farbstoffe und beschleunigen dadurch die Diffusion der Farbstoffe in den Fasern.

Gegenstand der vorliegenden Erfindung ist demnach auch ein Verfahren zum Veredeln von natürlichem oder synthetischem Fasermaterial mit oder ohne entsprechende Farbstoffe, wobei die Veredelung in Gegenwart der erfindungsgemässen nichtionogenen und/oder anionischen Styroloxidanlagerungsprodukte durchgeführt wird.

Die Einsatzmengen, in denen die erfindungsgemässen Styroloxidanlagerungsprodukte den Veredelungsflotten, wie z.B. Färbeflotten oder Vor- oder Nachbehandlungsflotten zugesetzt werden, bewegen sich, je nach Substrat, zweckmässigerweise zwischen 0,5 bis 20 g, vorzugsweise 1 bis 10 g, pro Liter Flotte.

Vorteilhafte Anwendungsformen der erfindungsgemässen Styroloxidanlagerungsprodukte bestehen darin, dass diese als nichtionogene und/oder anionische Produkte mit oder ohne Wasser in Kombination mit nichtionogenen oder anionischen Dispergatoren, Fettalkoholen, Fettsäureamiden, Alkylenbisfettsäureamiden, Alkenyldicarbonsäurealkylester, Metallstearaten, Siliconölen, z.B. -Dialkylpolysiloxanen, oder auch Mineralölen oder Alkanolaminen in stabilen, netzwirkenden und/oder schaumdämpfenden Formulierungen eingesetzt werden. Derartige Zübereitungen zeichnen sich zudem dadurch aus, dass sie fähig sind, wässrige Systeme praktisch vollkommen zu entlüften. Es können somit sowohl in den Applikationsbädern als auch in den Substraten Lufteinschlüsse vermieden werden. Durch eine solche Entlüftung können Fleckenbildung bei Färbungen und Ausrüstungen ausgeschlossen werden.

Bevorzugt sind wässerige oder wasserfreie Zübereitungen, welche, bezogen auf die gesamte Zübereitung,

(A) 2 bis 50 Gew.-% eines sauren Esters des Anlagerungsproduktes von Styroloxid an einen Polyalkylenglykolether der Formel (1),

(B) 5 bis 50 Gew.-% eines nichtionogenen Tensides, vorzugsweise eines aliphatischen Monoalkohols mit 6 bis 22 Kohlenstoffatomen oder eines Anlagerungsproduktes von 2 bis 80 Mol Ethylenoxid an 1 Mol eines aliphatischen Monoalkohols mit 6 bis 22 Kohlenstoffatomen oder eines Polyoxyethylenderivates eines Sorbitanfettsäureesters oder eines Styroloxidanlagerungsproduktes der Formel (2) oder Gemische davon und mindestens eine der folgenden Komponenten:

(C) 1 bis 30 Gew.-% eines Silikonöls z.B. eines Dialkylpolysiloxans wie Dimethylpolysiloxan,

(D) 10 bis 60 Gew.-% eines Mineralöls z.B. Paraffinöl wie Shell Oil L 6189 oder Mineralöle Esso 301-312,

(E) 20 bis 45 Gew.-% eines Dialkylesters einer ethylenisch ungesättigten aliphatischen Dicarbonsäure mit vorzugsweise 4 bis 12 Kohlenstoffatomen per Alkylrest, wie z.B. Maleinsäure-bis-2-ethylhexylester, Citracon-bis-2-ethylhexylester

(F) 10 bis 70 Gew.-% eines Diffusionsbeschleunigers, insbesondere eines aliphatischen oder aromatischen Carbonsäureesters wie Milchsäure-$C_4$-$C_{12}$-alkylester, Benzoesäure-$C_4$-$C_{12}$-alkylester, Benzoesäurephenylester oder Benzoesäurebenzylester, oder auch eines Alkylbenzols, wie Trimethylbenzol, Ethylbenzol,

(G) 0,5 bis 5 Gew.-% eines Salzes einer $C_{10}$-$C_{24}$-Fettsäure und eines mehrwertigen Metalles, wie z.B. Magnesiumdistearat, Calciumdibehenat oder Aluminiumtristearat und

(H) 0,5 bis 3 Gew.-% eines $C_1$-$C_4$-Alkylendiamids einer Fettsäure mit 10 bis 24 Kohlenstoffatomen, wie z.B. Methylen-bis-stearinsäureamid, Ethylen-bis-stearinsäureamid und Ethylen-bis-behensäureamid enthalten.

Weitere bevorzugte wässrige Zübereitungen bestehen darin, dass sie bezogen auf die gesamte Zübereitung,

(a) 15 bis 50 Gew.% eines nichtionischen Anlagerungsproduktes von Styroloxid an einen Polyalkylenglykolether der Formel (1), besonders eines Styroloxid-Anlagerungsproduktes der Formel (2),

(b) 1 bis 5 Gew.% eines sauren Esters oder dessen Salzes, z.B. Alkalimetall- oder Ammoniumsalz, einer Verbindung der Formel

(5) 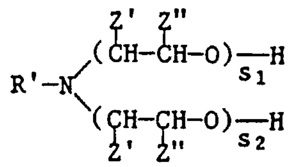

oder eines quaternierten Produktes des sauren Esters oder dessen Salzes, worin $R'$ einen Alkyl- oder Alkenylrest mit 12 bis 22 Kohlenstoffatomen, von $Z'$ und $Z''$ eines Wasserstoff, Methyl oder Phenyl und das andere Wasserstoff und $s_1$ und $s_2$ ganze Zahlen bedeuen, wobei die Summe $s_1 + s_2$ 2 bis 100 ist,

(c) 2 bis 10 Gew.% einer quaternären Ammoniumverbindung der Formel

(6) 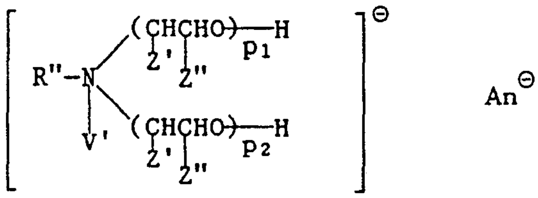

in der $R''$ einen Alkyl- oder Alkenylrest mit 12 bis 22 Kohlenstoffatomen, $V'$ einen gegebenenfalls substituierten Alkylrest, wie Methyl, Ethyl, Benzyl oder $-CH_2CONH_2$, von $Z'$ und $Z''$ eines Wasserstoff, Methyl oder Phenyl und das andere Wasserstoff, $An^{\ominus}$ ein Anion einer anorganischen oder organischen Säure und $p_1$ und $p_2$ je ganze Zahlen bedeuten, wobei die Summe von $p_1$ und $p_2$ 2 bis 100 ist, und gegebenenfalls zusätzlich mindestens eine der folgenden Komponenten:

(d) 0,5 bis 25 Gew.% eines Polyalkylenglykolethers der Formel

(7) $R'''\text{-O-}(\text{Alkylen-O})_q\text{-H}$

worin $R'''$ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen nichtionogenen Verbindung der Formel

(8) 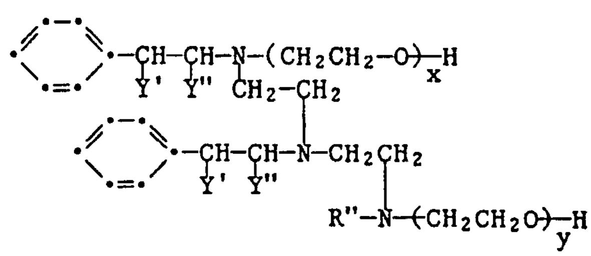

worin $R''$ einen Alkyl- oder Alkenylrest mit 12 bis 22 Kohlenstoffatomen, von $Y'$ und $Y''$ eines Phenyl und das andere Wasserstoff, und x und y ganze Zahlen bedeuten, wobei die Summe von x und y 80 bis 140 ist, enthält.

Diese Zübereitungen eignen sich insbesondere als Hilfsmittel beim Färben von natürlichen oder synthetischen Polyamidfasern, vor allem Wolle, mit anionischen Farbstoffen.

Nähere Angaben und bevorzugte Kombinationen und Anwendungsformen für Komponenten (b), (c), (d) und (e) können aus den Patentschriften DE-A-1 568 258, DE-A-1 619 385, EP-A-89004 und EP-A-312493 entnommen werden.

In den folgenden Herstellungs- und Anwendungsbeispielen beziehen sich die Prozentsätze, wenn nicht anders angegeben ist, auf das Gewicht; Teile sind Gewichtsteile. Die Mengen beziehen sich bei den Farbstoffen auf handelsübliche d.h. coupierte Ware und bei den Hilfsmitteln auf Reinsubstanz.

Herstellungsbeispiele

Beispiel 1:

163,8 g eines Anlagerungsproduktes von 4 Mol Ethylenoxid an 1 Mol $C_9$-$C_{11}$-Oxoalkohol (OH-Zahl: 171) werden mit 3,3 g Schwefelsäure (96 %) gemischt und auf 65° C aufgeheizt. Alsdann lässt man 60 g Styroloxid im Verlaufe von 35 Minuten zutropfen, wobei die Temperatur auf 87° C steigt. Anschliessend wird das Reaktionsprodukt 5 Stunden bei 75° C nachgerührt, mit Natriumbicarbonatlösung neutralisiert und filtriert. Das Filtrat wird eingeengt und der Rückstand getrocknet. Man erhält ein gelbliches, klares Produkt der Formel

(11) 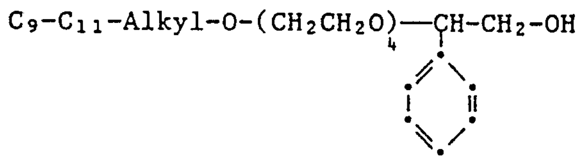

OH-Zahl: 127

Beispiel 2:

a) 350 g eines Anlagerungsproduktes von 35 Mol Ethylenoxid an 1 Mol Stearylalkohol (OH-Zahl: 32) werden mit 5,6 g Schwefelsäure (96 %) gemischt und auf 70° C erwärmt. Hierauf lässt man 24 g Styroloxid im Verlaufe von 30 Minuten bei 70-75° C zutropfen. Alsdann wird die Mischung 8 Stunden bei 75° C gerührt und anschliessend mit Natriumbicarbonatlösung neutralisiert und filtriert. Das Filtrat wird eingeengt und der Rückstand getrocknet. Man erhält ein bei Raumtemperatur wachsartiges Produkt der Formel

(12) 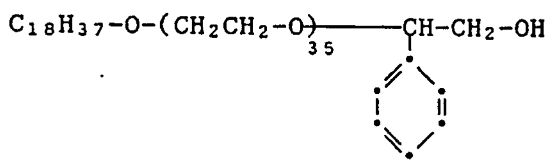

OH-Zahl: 30

b) 185 g des gemäss (a) hergestellten Produktes der Formel (12) werden auf 70° C erwärmt und mit 10 g Harnstoff vermischt. Nach 15 Minuten gibt man 10 g Sulfaminsäure zu und rührt 1 Stunde bei 80° C und 2 Stunden bei 95° C. Alsdann wird das Reaktionsprodukt mit 307,5 g Wasser verdünnt und bis zur vollständigen Lösung bei 65-70° C gerührt. Man erhält ein bei Raumtemperatur gelartiges Produkt, welches der Formel

(13) 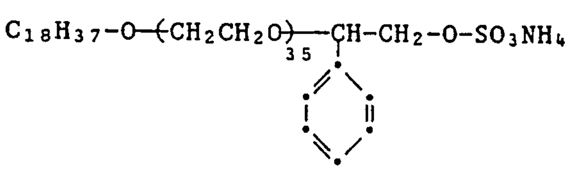

entspricht.

Beispiel 3:

112 g des gemäss Beispiel 2(a) hergestellten Styroloxidanlagerungsproduktes der Formel (12) werden mit 6,0 g Maleinsäureanhydrid langsam auf 70° C erwärmt und 1 Stunde bei dieser Temperatur gerührt. Hierauf wird die Reaktionsmischung weitere 3 Stunden bei 90° C gerührt. Man erhält ein bei Raumtemperatur wachsartiges Produkt der Formel

(14) 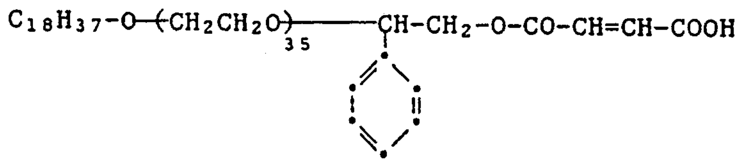

Säurezahl: 29

Beispiel 4:

105 g des Umsetzungsproduktes von 1 Mol $C_9$-$C_{11}$-Oxo-alkohol mit 6 Mol Ethylenoxid und 6 Mol Propylenoxid (OH-Zahl: 115) werden mit 1,9 g Schwefelsäure (96 %) gemischt und auf 65 °C erwärmt. Hierauf lässt man 25,8 g Styroloxid im Verlaufe von 15 Minuten zutropfen; dabei steigt die Temperatur auf 86 °C an. Alsdann wird das Reaktionsprodukt 5 Stunden bei 75 °C nachgerührt und anschliessend mit Natriumbicarbonat neutralisiert und filtriert. Das Filtrat wird eingeengt und der Rückstand getrocknet. Man erhält ein bräunliches, leicht trübes Produkt der Formel

(15) 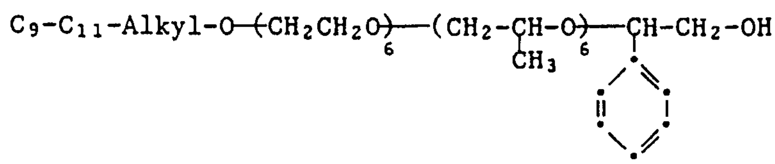

OH-Zahl: 96

Beispiel 5:

184 g eines Anlagerungsproduktes von 2 Mol Ethylenoxid an 1 Mol Isononylalkohol (OH-Zahl: 243) werden mit 4,2 g Schwefelsäure (96 %) gemischt und auf 65 °C aufgeheizt. Alsdann lässt man 96,0 g Styroloxid im Verlaufe von 60 Minuten zutropfen, wobei die Temperatur auf 81 °C steigt. Anschliessend wird das Reaktionsprodukt 5 Stunden bei 75 °C nachgerührt, mit Natriumbicarbonatlösung neutralisiert und filtriert. Das Filtrat wird eingeengt und der Rückstand getrocknet. Man erhält ein gelbliches, klares Produkt der Formel

(16) 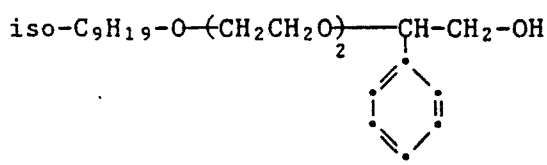

OH-Zahl: 159

Beispiel 6:

190 g eines Anlagerungsproduktes von 4 Mol Ethylenoxid an 1 Mol Isononylalkohol (OH-Zahl: 176) werden mit 3,9 g Schwefelsäure (96 %) gemischt und auf 65 °C aufgeheizt. Alsdann lässt man 72 g Styroloxid im Verlaufe von 60 Minuten zutropfen, wobei die Temperatur auf 80 °C steigt. Anschliessend wird das Reaktionsprodukt 5 Stunden bei 75 °C nachgerührt, mit Natriumbicarbonatlösung neutralisiert und filtriert. Das Filtrat wird am Rotationsverdampfer eingeengt und der Rückstand getrocknet. Man erhält ein gelbliches, klares Produkt der Formel

(17) 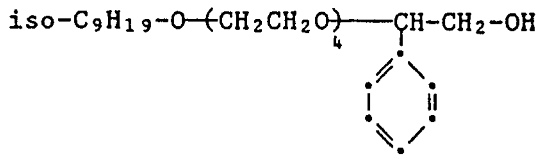

OH-Zahl: 119

Beispiel 7:

200,5 g eines Anlagerungsproduktes von 6 Mol Ethylenoxid an 1 Mol Isononylalkohol (OH-Zahl: 139,6) werden mit 3,9 g Schwefelsäure (96 %) gemischt und auf 65° C aufgeheizt. Alsdann lässt man 60 g Styroloxid im Verlaufe von 40 Minuten zutropfen, wobei die Temperatur auf 83° C steigt. Anschliessend wird das Reaktionsprodukt 5 Stunden bei 75° C nachgerührt, mit Natriumbicarbonatlösung neutralisiert und filtriert. Das Filtrat wird am Rotationsverdampfer eingeengt und der Rückstand getrocknet. Man erhält ein gelbliches, klares Produkt der Formel

(18) 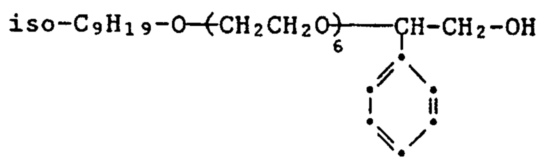

OH-Zahl 105

Beispiel 8:

a) 288,5 g eines Anlagerungsproduktes von 9 Mol Ethylenoxid an 1 Mol Tridecylalkohol (OH-Zahl: 97) werden mit 5,6 g Schwefelsäure (96 %) gemischt und auf 70° C erwärmt. Hierauf lässt man 60 g Styroloxid im Verlaufe von 30 Minuten bei 70-75° C zutropfen. Alsdann wird die Mischung 8 Stunden bei 75° C gerührt und anschliessend mit Natriumbicarbonatlösung neutralisiert und filtriert. Das Filtrat wird eingeengt und der Rückstand getrocknet. Man erhält ein bei Raumtemperatur wachsartiges Produkt der Formel

(19) 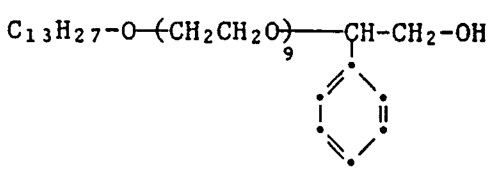

OH-Zahl: 78

b) 185 g des gemäss (a) hergestellten Produktes der Formel (19) werden auf 70° C erwärmt und mit 10 g Harnstoff vermischt. Nach 15 Minuten gibt man 10 g Sulfaminsäure zu und rührt 1 Stunde bei 80° C und 2 Stunden bei 95° C. Alsdann wird das Reaktionsprodukt mit 307,5 g Wasser verdünnt und bis zur vollständigen Lösung bei 65-70° C gerührt. Man erhält ein bei Raumtemperatur gelartiges Produkt, welches der Formel

(20) 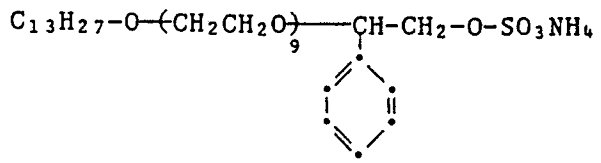

entspricht.

Beispiel 9:

818,5 g eines Anlagerungsproduktes von 20 Mol Ethylenoxid an 1 Mol Oleylalkohol (OH-Zahl: 52) werden mit 4,2 g Schwefelsäure (96 %) gemischt und auf 65 °C aufgeheizt. Alsdann lässt man 91 g Styroloxid im Verlaufe von 60 Minuten zutropfen, wobei die Temperatur auf 81 °C steigt. Anschliessend wird das Reaktionsprodukt 5 Stunden bei 75 °C nachgerührt, mit Natriumbicarbonatlösung neutralisiert und filtriert. Das Filtrat wird am Rotationsverdampfer eingeengt und der Rückstand getrocknet. Man erhält ein gelbliches, klares Produkt der Formel

(21) 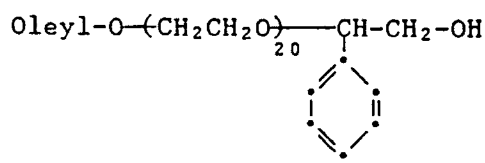

OH-Zahl: 47

Beispiel 10:

88,2 g des gemäss Beispiel 1 hergestellten Produktes der Formel (11) werden auf 70 °C erwärmt und mit 20,8 g Harnstoff vermischt. Nach 15 Minuten gibt man 20,6 g Sulfaminsäure zu und rührt 1 Stunde bei 80 °C und 2 Stunden bei 95 °C. Alsdann wird das Reaktionsprodukt mit 129,6 g Wasser verdünnt und bis zur vollständigen Lösung bei 65-70 °C gerührt. Man erhält ein bei Raumtemperatur gelartiges Produkt, welches der Formel

(22) 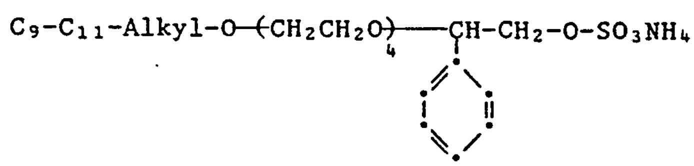

entspricht.

Auf ähnliche Art und Weise wie in den Beispielen 2, 3 und 10 beschrieben werden unter Verwendung der entsprechenden Ausgangsprodukte die folgenden anionischen Styroloxidanlagerungsprodukte der Formeln (23), (24), (25) und (26) hergestellt.

(23) 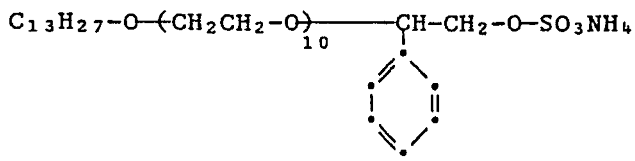

(24) 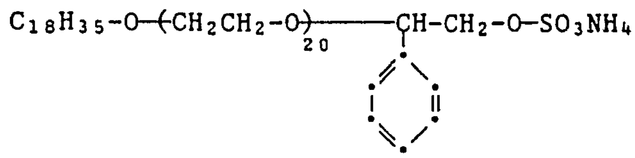

(25) 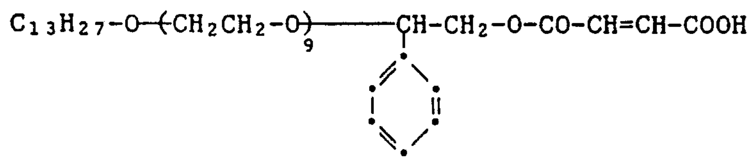

Säurezahl: 70,6

(26) 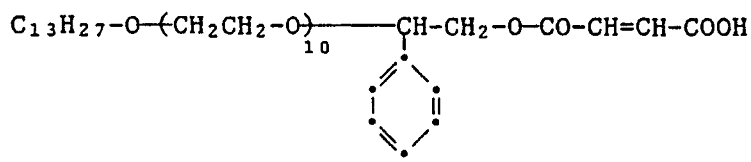

Säurezahl: 65

Beispiel 11:

361 g Ethylenglykolmonobutylether werden zusammen mit 1,84 g Bortrifluoridetherat auf 50 °C erwärmt. Hierauf lässt man innert 1 Stunde 240 g Styroloxyd zutropfen. Dabei steigt die Temperatur auf 85 °C. Alsdann rührt man 15 Minuten bei 85 °C und kühlt das Reaktionsgemisch auf Raumtemperatur. Nach Abdestillieren des überschüssigen Ethylenglykolmonobutyl ethers wird im Hochvakuum fraktioniert destilliert. Man erhält 230 g eines farblosen Produktes der folgenden Formel

(27) 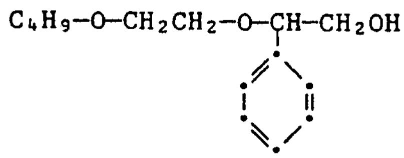

$Kp_{10}-2$: 118-120°C OH-Zahl: 252

Beispiel 12:

Zu 66,3 g des Anlagerungsproduktes der Formel (27) gemäss Beispiel 11 werden 14,2 g Phosphorpentoxid bei Raumtemperatur und unter starkem Rühren zugegeben. Dabei steigt die Temperatur auf 90 °C. Hierauf rührt man 4 Stunden bei Raumtemperatur. Man erhält ein klares und zähflüssiges Produkt, welches ein Gemisch aus den Verbindungen der Formeln

(28a) 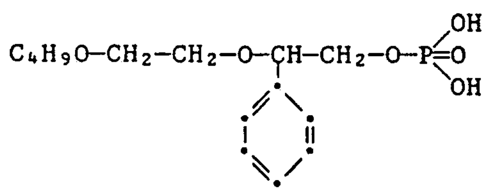

(28b) 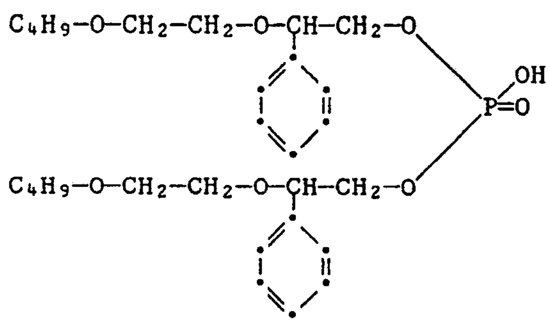

darstellt.

Auf ähnliche Art und Weise wie in den Beispielen 11 und 12 beschrieben werden unter Verwendung der entsprechenden Ausgangsprodukte die folgenden Styroloxidanlagerungsprodukte der Formeln (29), (30a) und (30b) hergestellt.

(29) 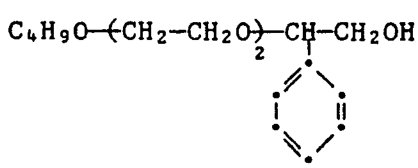

$Kp_{10}-2$: 68-70°C OH-Zahl: 194

Gemisch aus Mono- und Diphosphorsäureester der Formeln

(30a) 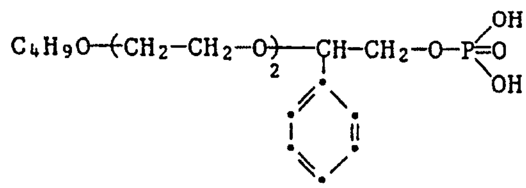

(30b) 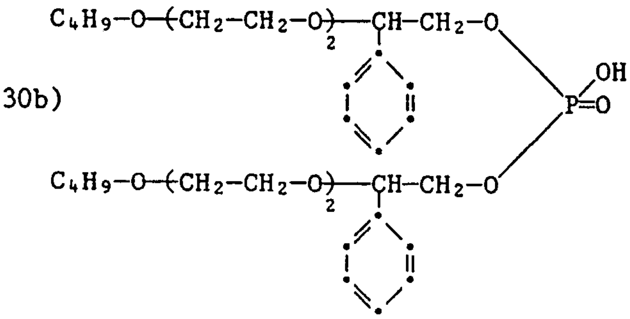

Beispiel 13:

508 g eines Anlagerungsproduktes von 3 Mol Propylenoxid und 5 Mol Ethylenoxyd an 1 Mol Dodecanol (OH-Zahl: 127.5) werden mit 9,7 g Methansulfonsäure gemischt und auf 70 °C erwärmt. Hierauf lässt man 138,8 g Styroloxyd im Verlaufe von 90 Minuten bei 70-80 °C zutropfen. Dann wird die Mischung 3 1/2 Stunden bei 80 °C gerührt und anschliessend mit Natriumbicarbonat-Lösung neutralisiert und filtriert. Das Filtrat wird eingeengt und der Rückstand getrocknet. Man erhält ein gelbliches, klares Produkt der Formel

(31) 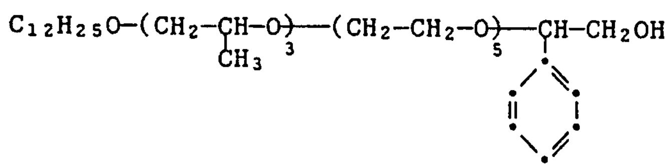

OH-Zahl: 90

Beispiel 14:

483 g eines Anlagerungsproduktes von 8 Mol Ethylenoxyd an 1 Mol $C_9$-$C_{11}$-Oxoalkohol (OH-Zahl: 116) werden mit 9 g Schwefelsäure (96 %) gemischt und auf 65 °C erwärmt. Alsdann lässt man 120 g Styroloxyd in 60 Minuten zutropfen, wobei die Temperatur auf 82 °C steigt. Anschliessend wird bei 75 °C 5 Stunden nachgerührt, mit Natriumbicarbonat-Lösung neutralisiert und filtriert. Das Filtrat wird eingeengt und der Rückstand getrocknet. Man erhält ein gelbliches, klares Produkt der Formel

(32) 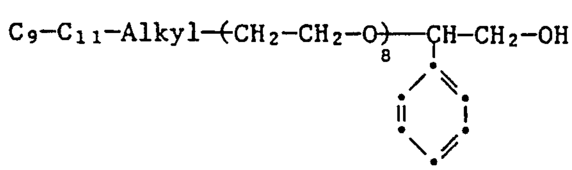

OH-Zahl: 91

Anwendungsbeispiele

Beispiel 15:

100 g Wollserge werden in einem Zirkulationsapparat bei 40° C während 15 Minuten mit einer Flotte aus 1,1 Liter Wasser eingenetzt, welche folgende Zusätze enthält:
3,2 g Essigsäure 80 %
5,0 g Natriumsulfat kalz.
1 g des gemäss Beispiel 1 hergestellten Styroloxidanlagerungsproduktes der Formel (11).

Alsdann werden 5 g des Farbstoffes Acid Black 172 C.I. 15711 zugegeben. Nach 10 Minuten wird die Färbeflotte im Verlaufe von 45 Minuten auf 85° C erwärmt und das Färbegut unter ständiger Zirkulation 60 Minuten bei dieser Temperatur gehalten. Anschliessend wird das Färbegut gespült und getrocknet. Man erhält ohne die konventionelle Erwärmung auf Kochtemperatur eine tiefe und echte schwarze Färbung der Wolle. Die Endflotte ist praktisch ungefärbt. Die Echtheiten entsprechen einer Färbung bei Kochtemperatur (98° C).

Beispiel 16:

In einem Zirkulationsfärbeapparat werden 100 kg Kreuzspulen aus Wolle mit einem Materialträger eingefahren. Im Ansatzbehälter werden 1200 l Wasser auf 60° C erwärmt und darin 1200 g eines wässrigen Präparates gelöst, welches bezogen auf das Präparat,
12 % Silikonöl z.B. Dimethylpolysiloxan
15 % 2-Ethyl-n-hexanol
15 % Paraffinöl
2 % eines wasserlöslichen, oberflächenaktiven Siloxanoxyalkylencopolymerisates
8 % des gemäss Beispiel 3 hergestellten Maleinsäurehalbesters und
2 % des gemäss Beispiel 9 hergestellten Styroloxidanlagerungsproduktes
enthält und mit Monoethanolamin auf pH 8 eingestellt ist. Dann wird die Flotte vom Ansatzbehälter durch das Material in den Färbeapparat gepumpt und anschliessend wechselseitig zirkuliert. Durch den Zusatz des Präparates tritt eine spontane Entlüftung des Färbesystems und damit eine gute Durchdringung des Färbegutes ein.

Hierauf gibt man der Flotte 2 kg des Farbstoffes der Formel

(101) 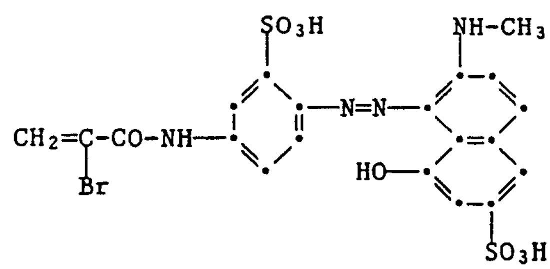

1 kg eines 1:1 Gemisches aus dem mit Chloracetamid quaternierten Anlagerungsproduktes von 7 Mol Ethylenoxid an 1 Mol eines $C_{16}$-$C_{18}$-Fettamingemisches und dem Ammoniumsalz des sauren Schwefelsäureesters des Anlagerungsproduktes von 7 Mol Ethylenoxid an 1 Mol eines $C_{16}$-$C_{18}$-Fettamingemisches (50%ige wässrige Zübereitung), erhitzt die Färbeflotte innerhalb 30 Minuten zum Sieden und färbt die Wolle 60 Minuten bei Siedetemperatur. Während der Färbung tritt praktisch keine Schaumentwicklung auf. Man erhält eine farbstarke und gleichmässige Durchfärbung der Kreuzspule.

Das in diesem Beispiel verwendete Präparat wird wie folgt hergestellt.

12 Teile Silikonöl werden in 15 Teilen 2-Ethyl-hexanol gelöst. Hierauf werden unter ständigem Rühren 15 Teile Paraffinöl, 2 Teile eines wasserlöslichen, oberflächenaktiven Siloxanoxyalkylencopolymerisates, 8 Teile des gemäss Beispiel 3 hergestellten Maleinsäurehalbesters, 2 Teile des gemäss Beispiel 9 hergestellten Styroloxidanlagerungsproduktes, 47,6 Teile Wasser und 0,4 Teile Monoethanolamin zugesetzt und 30 Minuten weitergerührt. Man erhält eine lagerstabile Formulierung mit netzender, entlüftender und besonders schaumdämpfender Wirkung.

Beispiel 17:

In einer Haspelkufe werden 100 kg Baumwollwirkware in 3000 l Wasser unter Zusatz von 3 kg eines wässrigen Präparates enthaltend, bezogen auf das Präparat,
7 % eines Silikonöls
11 % 2-Ethyl-hexanol und
11 % des gemäss Beispiel 10 hergestellten sauren Schwefelsäureesters genetzt. Innerhalb von 30 Sekunden ist die Baumwolle vollständig genetzt und entlüftet. Hierauf werden der Flotte 2 kg eines Farbstoffes der Formel

(102) 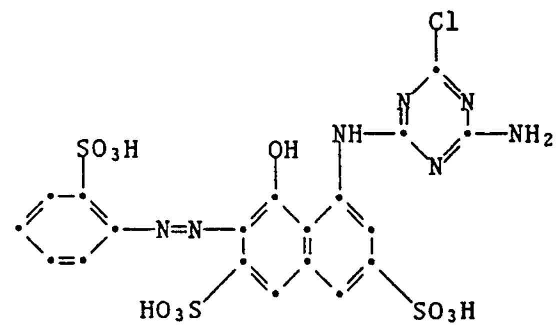

und die üblichen Chemikalien wie Elektrolyte und Alkalien zugesetzt, worauf die Ware zwei Stunden bei Siedetemperatur der Flotte gefärbt wird. Es tritt kein störendes Schäumen auf. Die gefärbte Ware ist gleichmässig und fleckenfrei durchgefärbt.

Das im Beispiel verwendete Präparat wird wie folgt hergestellt.

70 Teile Silikonöl werden zunächst in 110 Teilen 2-Ethyl-hexanol bei Raumtemperatur gelöst. Alsdann werden 110 Teile des gemäss Beispiel 10 hergestellten sauren Schwefelsäureesters und nach 30 Minuten 710 Teile Wasser zugegeben, worauf es noch 30 Minuten gerührt wird. Man erhält ein lagerstabiles Produkt mit ausgezeichneten netz- und entlüftenden Eigenschaften.

Beispiel 18:

Auf einem Kurzflotten-Jet werden 100 kg Baumwolltricot in 600 Liter Wasser bei 40 °C eingenetzt. In die Flotte gibt man 36 kg Natriumchlorid, 5 kg des Farbstoffes der Formel (102) sowie 0,5 kg einer Zübereitung, enthaltend
186,75 g Mineralöl, (z.B. Shell Oil L 6189),
185 g Maleinsäure-bis-2-ethylhexylester,
10 g Magnesium-distearat
8,25 g N,N-Ethylenbisstearamid
55 g eines Polyoxyethylenderivates von Sorbitantristearat mit 20 Oxyethyleneinheiten z.B. Tween 65
55 g des gemäss Beispiel 8 hergestellten sauren Schwefelsäureesters.

Man färbt die Ware während 45 Minuten bei 40 °C. Dann erfolgt der Zusatz von 0,6 kg kalziniertem Natriumcarbonat und nach weiteren 5 Minuten von 1,2 kg einer wässrigen 36 %igen Natriumhydroxydlösung. Hierauf wird das Tricot während weiteren 40 Minuten gefärbt, anschliessend gespült und nachgewaschen. Man erhält eine echte, egale Rotfärbung des Tricots. Während des Färbeprozesses tritt kein Schäumen und keine Störung des Warenlaufes auf.

Die in diesem Beispiel verwendete Zübereitung wird wie folgt hergestellt.

186,75 g Mineralöl, 185 g Maleinsäure-bis-2-ethylhexylester, 10 g Magnesiumdistearat und 8,25 g N,N-Ethylen-bis-stearamid werden unter ständigem Rühren auf 110 °C erwärmt bis eine klare Lösung eintritt. Man kühlt während 5 Minuten auf 45 °C ab und verteilt in der Lösung 55 g eines Polyoxyethylenderivates von Sorbitantristearat mit 20 Oxyethyleneinheiten z.B. Tween 65 und 55 g des gemäss Beispiel 8 hergestellten sauren Schwefelsäureesters. Man erhält eine stabile Formulierung, die insbesondere als Schaum-dämpfungsmittel in alkalischen Flotten und bei hohen Scherkräften sehr wirksam ist.

Beispiel 19:

100 Teile eines Polyestergewebes werden in eine 60 °C warme Färbeflotte gegeben, die 1300 Teile

Wasser, 2 g/l Ammoniumsulfat, 2,5 Teile eines Farbstoffes der Formel

(103) 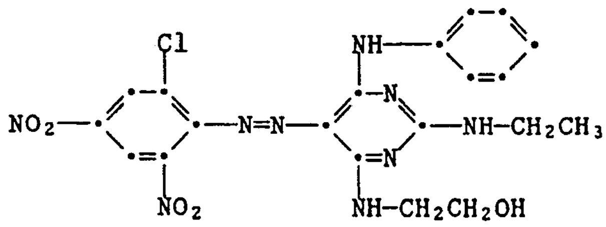

und 2 Teile einer Hilfsmittelformulierung bestehend aus
16 Teilen des gemäss Beispiel 3 hergestellten Maleinsäurehalbesters
24 Teilen des Anlagerungsproduktes von 18 Mol Ethylenoxid an 1 Mol eines $C_{12}$-$C_{18}$-Fettalkoholgemisches und
60 Teilen Benzoesäurebenzylester
enthält und mit Ameisensäure auf pH 5 gestellt ist. Man steigert die Temperatur der Flotte in Verlauf von 30 Minuten auf 130 °C und färbt 60 Minuten bei dieser Temperatur.

Danach wird die Flotte auf 70 °C abgekühlt, abgelassen und die Ware gespült. Man erhält ohne die übliche reduktive Nachreinigung eine egale und reibechte rote Färbung mit einer hohen Farbstoffausbeute.

Die in diesem Beispiel verwendete Hilfsmittelformulierung wird wie folgt hergestellt:

In einem heizbaren Rührgefäss werden 60 Teile Benzoesäurebenzylester auf 60 °C unter ständigem Rühren erwärmt und anschliessend 24 Teile des Anlagerungsproduktes von 18 Mol Ethylenoxid an 1 Mol eines $C_{12}$-$C_{19}$-Fettalkoholgemisches und 16 Teile des gemäss Beispiel 3 hergestellten Maleinsäurehalbesters eingerührt. Man erhält nach Abkühlen auf Raumtemperatur eine lagerstabile Formulierung, die insbesondere beim Färben von Polyesterfasern geeignet ist.

Beispiel 20:

100 Teile eines Wickelkörpers aus texturierten Polyestergarnen werden in einen HT-Färbeapparat, welcher 800 Teile 40 °C warmes Wasser, 2 Teile Ammoniumsulfat, 4 Teile eines Farbstoffes der Formel

(104) 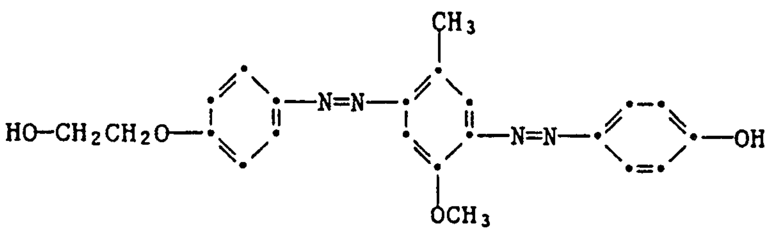

und 2 Teile der Hilfsmittelformulierung gemäss Beispiel 19 enthält und dessen Flotte mit Ameisensäure auf pH 5,5 gestellt ist, eingebracht.

Danach wird die Temperatur der Flotte in Verlaufe von 40 Minuten auf 128 °C erhöht und die Ware 60 Minuten bei dieser Temperatur gefärbt.

Während der Aufheizphase wird im Materialblock kein Anstieg des Differenzdruckes zwischen innen und aussen festgestellt. Anschliessend wird die Flotte auf 70 °C abgekühlt und das Substrat wie üblich reduktiv gereinigt, gespült und getrocknet. Man erhält eine farbstarke und egale orange Färbung, die sich durch gute Durchfärbung und gute Echtheiten auszeichnet.

Beispiel 21:

100 g Wollserge werden in einem Zirkulationsapparat z.B. Ahiba-Turbomat bei 40 °C während 15 Minuten mit einer Flotte aus 1,1 Liter Wasser eingenetzt, welche folgende Zusätze enthält:
3,2 g Essigsäure 80 %

5 g Natriumsulfat kalz.
2 g einer wässrigen Zübereitung enthaltend, bezogen auf diese Zübereitung, 30 Gew.% des gemäss Beispiel 1 hergestellten Styroloxidanlagerungsproduktes der Formel (11),
10 Gew.% des Hilfsmittelgemisches $A_1$ gemäss EP-A-0089004 und
20 Gew.% Triethylenglykolmonobutylether

Alsdann werden 4 g des Farbstoffes Acid Black 172 CI. 15711 zugegeben. Nach 10 Minuten wird die Färbeflotte im Verlaufe von 50 Minuten auf 90 °C erwärmt und das Färbegut unter ständiger Zirkulation 60 Minuten bei dieser Temperatur gehalten. Anschliessend wird das Färbegut gespült und getrocknet.

Man erhält eine tiefe, egale und echte schwarze Färbung der Wolle. Das Färbebad ist zu 95 % erschöpft, obwohl bei 90 °C gefärbt wird. Die Echtheiten entsprechen einer konventionellen Färbung bei Kochtemperatur (98 °C).

## Ansprüche

1. Anlagerungsprodukte von Styroloxid an Polyalkylenglykolether der Formel
(1) $R\text{-}O\text{-}(\text{Alkylen-}O)_m\text{-}H$
ihre saure Ester und deren Salze, worin
R einen aliphatischen Rest mit mindestens 4 Kohlenstoffatomen bedeutet,
"Alkylen" für einen Alkylenrest von 2 oder 3 Kohlenstoffatomen steht und
m eine Zahl von 1 bis 100 ist.

2. Styroloxidanlagerungsprodukte gemäss Anspruch 1, dadurch gekennzeichnet, dass in Formel (1) R den Kohlenwasserstoffrest eines ungesättigten oder gesättigten aliphatischen Alkohols mit 6 bis 24 Kohlenstoffatomen bedeutet.

3. Styroloxidanlagerungsprodukte gemäss einem der Ansprüche 1 und 2, dadurch gekennzeichnet, dass in Formel (1)
R einen Alkyl- oder Alkenylrest von 8 bis 22 Kohlenstoffatomen bedeutet.

4. Styroloxidanlagerungsprodukte gemäss einem der Ansprüche 1 bis 3 dadurch gekennzeichnet, dass in Formel (1)
"Alkylen" Ethylen bedeutet.

5. Styroloxidanlagerungsprodukte gemäss einem der Ansprüche 1 bis 4, dadurch gekennzeichnet, dass in Formel (1) m 2 bis 100 ist.

6. Styroloxidanlagerungsprodukte gemäss einem der Ansprüche 1 bis 5, dadurch gekennzeichnet, dass in Formel (1)
m 4 bis 80, vorzugsweise 4 bis 40 ist.

7. Styroloxidanlagerungsprodukte gemäss Anspruch 1 dadurch gekennzeichnet, dass sie der Formel

(2) 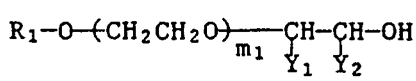

entsprechen, worin
$R_1$ Alkyl oder Alkenyl je mit 8 bis 22 Kohlenstoffatomen,
von $Y_1$ und $Y_2$ eines Phenyl und das andere Wasserstoff und
$m_1$ 4 bis 80 bedeuten.

8. Styroloxidanlagerungsprodukte gemäss Anspruch 7, dadurch gekennzeichnet, dass in Formel (2) $R_1$ $C_9$-$C_{11}$-Alkyl, Tridecyl, Oleyl oder Stearyl und $m_1$ 4 bis 35 bedeuten.

9. Saure Ester von Styroloxidanlagerungsprodukten gemäss Anspruch 1 dadurch gekennzeichnet, dass sie der Formel

(3) 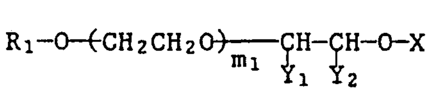

entsprechen, worin
$R_1$ Alkyl oder Alkenyl je mit 8 bis 22 Kohlenstoffatomen,
von $Y_1$ und $Y_2$ eines Phenyl und das andere Wasserstoff,

X den Maleinsäure-, Sulfobernsteinsäure-, Schwefelsäure- oder Phosphorsäurerest und $m_1$ 4 bis 80 bedeuten.

10. Saure Ester von Styroloxidanlagerungsprodukten gemäss Anspruch 9, dadurch gekennzeichnet, dass in Formel (3) $R_1$ $C_9$-$C_{11}$-Alkyl, Tridecyl, Stearyl oder Oleyl, X den Maleinsäure-, Phosphorsäure- oder Schwefelsäurerest und $m_1$ 4 bis 35 bedeuten.

11. Saure Ester von Styroloxidanlagerungsprodukten gemäss Anspruch 1 dadurch gekennzeichnet, dass sie der Formel

(4) 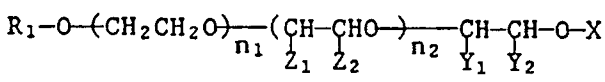

entsprechen, worin
$R_1$ Alkyl oder Alkenyl je mit 8 bis 22 Kohlenstoffatomen,
von $Y_1$ und $Y_2$ eines Phenyl und das andere Wasserstoff,
von $Z_1$ und $Z_2$ eines Methyl und das andere Wasserstoff,
X den Maleinsäure-, Sulfobernsteinsäure-, Schwefelsäure- oder Phosphorsäurerest und die Summe von $n_1 + n_2$ 4 bis 30, vorzugsweise 6 bis 18 bedeuten.

12. Wässerige oder wasserfreie Zübereitung, welche, bezogen auf die gesamte Zübereitung,
(A) 2 bis 50 Gew.-% eines sauren Esters des Anlagerungsproduktes von Styroloxid an einen Polyalkylenglykolether der Formel
(1) $R\text{-}O\{Alkylen\text{-}O\}_m\text{-}H$
worin
R einen aliphatischen Rest mit mindestens 6 Kohlenstoffatomen bedeutet,
"Alkylen" für einen Alkylenrest von 2 oder 3 Kohlenstoffatomen steht und
m eine Zahl von 2 bis 100 ist,
(B) 5 bis 50 Gew.-% eines nichtionogenen Tensides und mindestens eine der folgenden Komponenten
(C) 1 bis 30 Gew.-% eines Silikonöls,
(D) 10 bis 60 Gew.-% eines Mineralöls,
(E) 20 bis 45 Gew.-% eines Dialkylesters einer ethylenisch ungesättigten aliphatischen Dicarbonsäure,
(F) 10 bis 70 Gew.-% eines Diffusionsbeschleunigers, insbesondere eines aliphatischen oder aromatischen Carbonsäureesters oder eines Alkylbenzols,
(G) 0,5 bis 5 Gew.-% eines Salzes einer $C_{10}$-$C_{24}$-Fettsäure und eines mehrwertigen Metalls und
(H) 0,5 bis 3 Gew.-% eines $C_1$-$C_4$-Alkylendiamids einer Fettsäure mit 10 bis 24 Kohlenstoffatomen enthält.

13. Wässerige Zübereitung, welche, bezogen auf die gesamte Zübereitung,
(a) 15 bis 50 Gew.% eines nichtionischen Anlagerungsproduktes von Styroloxid an einen Polyalkylenglykolether der Formel
(1) $R\text{-}O\{Alkylen\text{-}O\}_m\text{-}H$
worin
R einen aliphatischen Rest mit mindestens 6 Kohlenstoffatomen bedeutet,
"Alkylen" für einen Alkylenrest von 2 oder 3 Kohlenstoffatomen steht und
m eine Zahl von 2 bis 100 ist,
(b) 1 bis 5 Gew.% eines sauren Esters oder dessen Salzes einer Verbindung der Formel

(5) 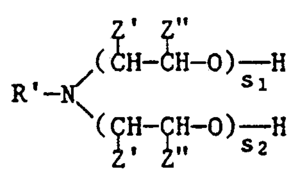

oder eines quaternierten Produktes des sauren Esters oder dessen Salzes, worin $R'$ einen Alkyl- oder Alkenylrest mit 12 bis 22 Kohlenstoffatomen, von $Z'$ und $Z''$ eines Wasserstoff, Methyl oder Phenyl und das andere Wasserstoff und $s_1$ und $s_2$ ganze Zahlen bedeuen, wobei die Summe $s_1 + s_2$ 2 bis 100 ist,
(c) 2 bis 10 Gew.% einer quaternären Ammoniumverbindung der Formel

(6) 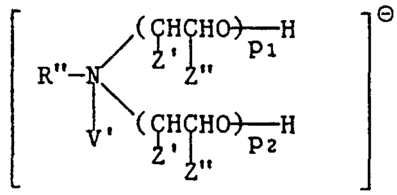 $An^{\ominus}$

in der R″ einen Alkyl- oder Alkenylrest mit 12 bis 22 Kohlenstoffatomen, V′ einen gegebenenfalls substituierten Alkylrest, von Z′ und Z″ eines Wasserstoff, Methyl oder Phenyl und das andere Wasserstoff, $An^{\ominus}$ ein Anion einer anorganischen oder organischen Säure und $p_1$ und $p_2$ ganze Zahlen bedeuten, wobei die Summe von $p_1$ und $p_2$ 2 bis 100 ist, und gegebenenfalls zusätzlich mindestens eine der folgenden Komponenten

(d) 0,5 bis 25 Gew.% eines Polyalkylenglykolethers der Formel

(7) $R'''\text{-O}(\text{Alkylen-O})_q\text{-H}$

worin R‴ einen Alkyl- oder Alkenylrest mit 4 bis 22 Kohlenwasserstoffatomen bedeutet, "Alkylen" für den Ethylenrest oder Propylenrest steht und q 2 bis 85 ist, und

(e) 0,5 bis 5 Gew.% einer stickstoffhaltigen nichtionogenen Verbindung der Formel

(8) 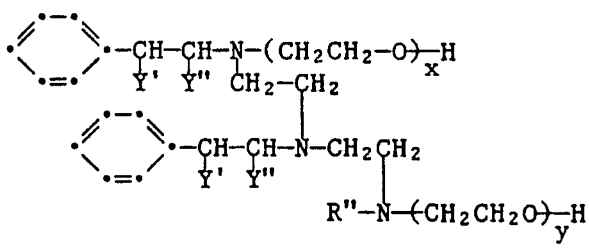

worin R″ einen Alkyl- oder Alkenylrest mit 12 bis 22 Kohlenstoffatomen, von Y′ und Y″ eines Phenyl und das andere Wasserstoff, und x und y ganze Zahlen bedeuten, wobei die Summe von x und y 80 bis 140 ist, enthält.

14. Verwendung der Styroloxidanlagerungsprodukte gemäss einem der Ansprüche 1 bis 11 oder der Zübereitung gemäss Anspruch 12 oder 13 beim Veredeln von natürlichem oder synthetischem Fasermaterial, insbesondere von natürlichen oder synthetischen Polyamidfasern, Polyesterfasern oder Cellulosefasern mit oder ohne entsprechende Farbstoffe.

15. Verfahren zum Veredeln von natürlichem oder synthetischem Fasermaterial, insbesondere von natürlichen oder synthetischen Polyamidfasern, Polyesterfasern oder Cellulosefasern, mit oder ohne entsprechende Farbstoffe, dadurch gekennzeichnet, dass die Veredelung in Gegenwart von Styroloxidanlagerungsprodukten gemäss Anspruch 1 der Formel

1(a) 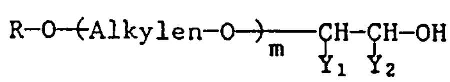

oder ihrer sauren Ester oder deren Salze, worin
R einen aliphatischen Rest mit mindestens 4 Kohlenstoffatomen bedeutet,
"Alkylen" für einen Alkylenrest von 2 oder 3 Kohlenstoffatomen steht und
m eine Zahl von 1 bis 100 bedeutet, durchgeführt wird.

Europäisches Patentamt

# EUROPÄISCHER RECHERCHENBERICHT

Nummer der Anmeldung

EP 89 81 0955

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 197 001 (CIBA-GEIGY AG)<br>* Patentanspruch 1; Seite 14, Beispiel 1 *<br>--- | 1-15 | C 08 G 65/32<br>C 08 G 65/26<br>D 06 P 1/613 |
| Y | EP-A-0 197 005 (CIBA-GEIGY AG)<br>* Seite 1, Formel 1; Beispiele *<br>--- | 1-15 | |
| Y,D | EP-A-0 089 004 (CIBA-GEIGY AG)<br>* Patentansprüche *<br>--- | 1-15 | |
| A,D | DE-A-1 619 385 (CIBA-GEIGY AG)<br>* Patentansprüche *<br>----- | 1 | |
| | | | **RECHERCHIERTE SACHGEBIETE (Int. Cl.5)** |
| | | | C 08 G<br>D 06 M<br>D 06 P |

Der vorliegende Recherchenbericht wurde für alle Patentansprüche erstellt

| Recherchenort | Abschlußdatum der Recherche | Prüfer |
|---|---|---|
| DEN HAAG | 02-04-1990 | PRAS J-L.C.N. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)